# EUROPÄISCHE PATENTSCHRIFT

(11) **EP 3 298 921 B1**
(45) Veröffentlichungstag und Bekanntmachung des Hinweises auf die Patenterteilung: **20.11.2019**
(21) Anmeldenummer: 17186908.4
(22) Anmeldetag: 18.08.2017
(51) Int. Cl.: A43B 3/00, A43B 17/00, A43B 13/14, A43B 13/12

(54) **EINLEGESOHLE ODER SCHUHSOHLE**
INSOLE OR SHOE SOLE
SEMELLE INTÉRIEURE DE CHAUSSURE OU SEMELLE DE CHAUSSURE

(30) Priorität: 27.09.2016 AT 4432016
(43) Veröffentlichungstag der Anmeldung: 28.03.2018
(73) Patentinhaber: stAPPtronics GmbH, 6832 Sulz (AT)
(72) Erfinder: Krimmer, Peter, 1130 Wien (AT); Olbrich, Philip, 1220 Wien (AT); Fröis, Thomas, 6832 Sulz (AT)
(74) Vertreter: Hofmann, Ralf U.

(56) Entgegenhaltungen:
- US-A1- 2012 291 564
- US-A1- 2013 213 146
- US-A1- 2015 330 855
- US-B1- 6 195 921
- US-B1- 6 216 545

## Beschreibung

Die Erfindung bezieht sich auf eine Einlegesohle oder Schuhsohle, welche eine Sensoreinrichtung mit mehreren Sensorfeldern zur Druckerfassung aufweist.

Mit solchen Einlegesohlen bzw. Schuhsohlen lassen sich u.a. Bewegungsabläufe eines Nutzers erfassen und auswerten, beispielsweise um Fehlbelastungen festzustellen. Hierzu können der Druck auf die einzelnen Sensorfelder und der zeitliche Druckverlauf erfasst und ausgewertet werden.

Aus der DE 10 2006 025 447 A1 geht eine Einlegesohle hervor, welche mehrere Sensoren aufweist. Ein jeweiliger Sensor kann ein piezoresistives Material aufweisen oder auch als kapazitiver Sensor ausgebildet sein. Ebenfalls in die Einlegesohle integriert kann eine Elektronikeinheit sein, welche mit den Sensoren elektrisch verbunden ist und die von den Sensoren ausgegebenen Signale auswertet. Eine ähnliche Einlegesohle geht aus der DE 10 201 134 A1 hervor.

Aus der DE 10 2012 004 117 A1 geht eine als Einlegesohle oder Schuhsohle ausgebildete Einrichtung hervor, welche eine Sensoreinrichtung mit mehreren Sensorfeldern zur Druckerfassung und eine Elektronikeinheit mit elektronischen Bauteilen aufweist, die mit den Sensorfeldern elektrisch verbunden sind. Die Elektronikeinheit ist eine Starrflexplatine, die mit den elektronischen Bauteilen bestückt ist. Die Sensorfelder der Sensoreinrichtung sind als kapazitive Sensoren ausgebildet. Hierzu weist die Sensoreinrichtung eine erste Kondensatorplattenseite, die als textile Sensorschicht ausgebildet ist, und eine zweite Kondensatorplattenseite auf, die von einer elektrisch leitenden Ground-Schicht gebildet wird. Die beiden Kondensatorplattenseiten sind durch ein verformbares Dielektrikum getrennt. Die textile Sensorschicht weist zur Ausbildung der Sensorfelder mehrere leitende Bereiche auf, welche über Sensorzuleitungen mit Sensoranschlusstorten verbunden sind, die in mittleren Bereichen der textilen Sensorschicht angeordnet sind. Die Sensoranschlusstorten liegen an Anschlusstorten der Platine an.

Aus der EP 3 047 794 A1 geht ein textiler piezoresistiver Sensor zur Erfassung von Herzschlag und Atmung hervor, der sich insbesondere zur Integration in Überzüge von Betten, Stühlen oder Fahrzeugsitzen eignet. Der Sensor umfasst eine erste textile Lage, auf welche Streifen aus leitfähigem Material durch Aufdrucken, Weben oder Sticken aufgebracht sind, eine in analoger Weise ausgebildete zweite Lage und eine zwischen diesen beiden Lagen angeordnete Lage aus einem piezoresistiven Material.

Die aus der US 2015/3330855 A1 hervorgehende Einlegesohle weist eine mehrlagig ausgebildete Sensoreinrichtung mit elektrisch leitfähigen Lagen auf, zwischen denen ein piezoresistives Material angeordnet ist, wobei ein einzelnes durchgehendes Sensorfeld ausgebildet wird. Eine außerhalb der Schuhsohle angeordnete Elektronikeinheit ist mit der Sensoreinrichtung über eine Kabelverbindung verbunden.

Aus der US 2012/0291564 A1 geht ein Schuh hervor, in dessen Schuhsohle eine Sensoreinrichtung mit mehreren Sensorfeldern und eine Elektronikeinheit integriert sind. Die Sensoreinrichtung weist eine Lage mit Elektrodenpaaren auf, welche an einer Lage eines kraftabhängigen Widerstandselements anliegen. Die Elektronikeinheit ist im Bereich der Mitte der Sohle angeordnet und mit der Sensoreinrichtung über eine Steckverbindung verbunden.

Eine Schuhsohle mit einer Sensoreinrichtung geht aus der US 6,216,545 B1 hervor. Die Sensoreinrichtung umfasst eine mittlere Lage aus einem piezoresistiven Material sowie obere und untere Lagen, die im Bereich eines jeweiligen Sensorfeldes jeweils ein elektrisch leitfähiges Material aufweisen. Die obere und untere Lage weisen zur elektrischen Verbindung mit einer Elektronikeinheit abstehende flexible Laschen mit Leiterbahnen auf. Die Leiterbahnen werden von einem Stecker der Elektronikeinheit kontaktiert, in den die Enden der Laschen eingesteckt werden.

Aufgabe der Erfindung ist es, eine vorteilhafte Einlegesohle oder Schuhsohle der eingangs genannten Art bereitzustellen, welche sich durch einen einfachen Aufbau auszeichnet und einfach herstellbar ist. Erfindungsgemäß gelingt dies durch eine Einlegesohle bzw. Schuhsohle mit den Merkmalen des Anspruchs 1.

Bei der Einlegesohle oder Schuhsohle gemäß der Erfindung weist die Sensoreinrichtung eine Sandwichstruktur mit einer mittleren Lage aus einem piezoresistiven Material, einer oberhalb der mittleren Lage angeordneten oberen Lage und einer unterhalb der mittleren Lage angeordneten unteren Lage auf, wobei die obere und untere Lage im Bereich eines jeweiligen Sensorfeldes jeweils ein elektrisch leitfähiges Material aufweisen. Auf diese Weise werden also piezoresistive Sensorfelder ausgebildet. Zur elektrischen Verbindung der Sensorfelder mit der Elektronikeinheit weisen die obere und die untere Lage jeweils eine abstehende flexible Lasche mit Leiterbahnen auf und die Elektronikeinheit weist Kontaktbereiche auf, an welchen die Laschen mit ihren Leiterbahnen anliegen.

Durch die Ausbildung der Schuhsohle mit piezoresistiven Sensoren wird eine einfache Auswertung der Messsignale ermöglicht. Da piezoresistive Sensoren durch die Sensorfelder der Sensoreinrichtung ausgebildet werden, welche die mittlere Lage, obere Lage und untere Lage umfasst, in Verbindung mit den abstehenden flexiblen Laschen, welche die obere und die untere Lage aufweisen, wird ein einfacher Aufbau sowie eine einfache Herstellbarkeit erreicht. Zur elektrischen Verbindung der Sensorfelder mit den elektrischen Bauteilen der Elektronikeinheit werden Leiterbahnen, mit welchen die Laschen versehen sind, an Kontaktbereiche der Elektronikeinheit angelegt.

Die an der oberen und unteren Lage angeordneten Laschen sind im mit der Elektronikeinheit verbundenen Zustand günstigerweise im Bereich, in welchem sie von der jeweiligen Lage ausgehen, umgebogen oder umgeknickt. Vorteilhafterweise können die Kontaktbereiche für die Leiterbahnen der unteren Lasche an der Oberseite der Elektronikeinheit und die Kontaktbereiche für die Leiterbahnen der oberen Lasche an der Unterseite der Elektronikeinheit angeordnet sein. Zum Andrücken der Leiterbahnen an die Kontaktbereiche der Elektronikeinheit können in einer vorteilhaften Ausführungsform Klemmelemente, beispielsweise in der Form von federelastischen Zungen, vorgesehen sein. Stattdessen oder alternativ könnten die Laschen auch an die Elektronikeinheit angeklebt sein.

Zur Erleichterung der Positionierung einer jeweiligen Lasche kann diese mindestens ein Loch aufweisen, durch welches im mit der Elektronikeinheit verbundenen Zustand ein Positionierungsstift ragt. Der mindestens eine Positionierungsstift kann hierbei insbesondere am Klemmelement angeordnet sein.

Das elektrisch leitfähige Material zur Ausbildung eines jeweiligen Sensorfeldes wird in einer bevorzugten Ausführungsform der Erfindung durch ein auf ein Grundmaterial der oberen bzw. unteren Lage aufgesticktes elektrisch leitfähiges Garn ausgebildet. Vorteilhaft ist es hierbei, wenn auch die Leiterbahnen der Laschen von einem solchen aufgestickten elektrisch leitfähigen Garn gebildet werden. Auch die Sensorzuleitungen, welche die Leiterbahnen der Laschen fortsetzen und mit den Sensorfeldern verbinden, können von einem aufgestickten elektrisch leitfähigen Garn gebildet werden. Besonders vorteilhaft ist es, wenn alle elektrisch leitfähigen Teile der oberen und unteren Lage von einem aufgestickten elektrisch leitfähigen Garn gebildet werden.

In einer alternativen Ausführungsform der Erfindung wäre es aber auch denkbar und möglich, dass das elektrisch leitfähige Material im Bereich eines jeweiligen Sensorfeldes und/oder die Leiterbahnen der Laschen und/oder die Sensorzuleitungen von einer elektrisch leitfähigen Beschichtung gebildet werden.

Weitere Vorteile und Einzelheiten der Erfindung werden im Folgenden anhand der beiliegenden Zeichnung erläutert. In dieser zeigen:
Fig. 1 eine Schrägsicht einer Einlegesohle gemäß der Erfindung;
Fig. 2 eine schematische Explosionsdarstellung;
Fig. 3 eine Seitenansicht (das Sohlenbasisteil durchsichtig dargestellt);
Fig. 4 einen Schnitt entlang der Linie AA von Fig. 3;
Fig. 5 ein vergrößertes Teil B von Fig. 4;
Fig. 6 eine Draufsicht auf die untere Lage der Sensoreinrichtung;
Fig. 7 eine Draufsicht auf die obere Lage der Sensoreinrichtung (das Grundmaterial durchsichtig dargestellt);

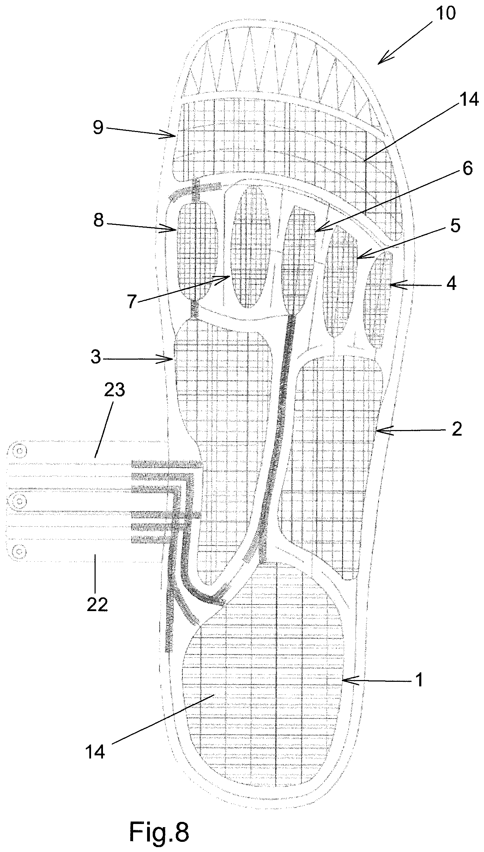

Fig. 8 eine Draufsicht auf die Sensoreinrichtung (das Grundmaterial der oberen Lage und die mittlere Lage durchsichtig dargestellt).

Ein Ausführungsbeispiel einer erfindungsgemäßen Einlegesohle für einen Schuh ist in den (teilweise vereinfachten und schematisierten) Figuren dargestellt. Die Einlegesohle weist eine Sensoreinrichtung 10 auf, die mehrere Sensorfelder 1-9 besitzt, von denen jeweils ein auf dem jeweiligen Sensorfeld 1-9 lastender Druck erfassbar ist. Die Sensoreinrichtung weist sandwichartig übereinander liegende Lagen 11-13 auf.

Die mittlere Lage 12 besteht aus einem piezoresistiven Material, d.h. der Durchgangswiderstand durch die mittlere Lage 12 (also rechtwinkelig zur Ebene der mittleren Lage 12) hängt von der Druckbelastung der mittleren Lage 12 ab. Beispielsweise kann es sich um ein piezoresistives Vlies handeln. Z.B. kann ein solches piezoresistives Vlies in Form eines Polyestervlieses ausgebildet sein, welches mit einem elektrisch leitfähigen Material, insbesondere einem Metall oder Carbon, beschichtet ist. Derartige piezoresistive Materialien sind bekannt.

Die untere und obere Lage 11, 13 weisen im Bereich 1a-9a; 1b-9b eines jeweiligen Sensorfeldes 1-9 jeweils ein elektrisch leitfähiges Material 14 auf. Dieses elektrisch leitfähige Material 14 wird im Ausführungsbeispiel von einem elektrisch leitfähigen Garn gebildet, mit welchem das Basismaterial der unteren bzw. oberen Lage 11, 13 bestickt ist. Im jeweiligen Bereich 1a-9a; 1b-9b wird vom aufgestickten leitfähigen Garn jeweils eine gitterförmige Struktur ausgebildet. Ein jeweiliger Bereich 1a-9a; 1b-9b könnte auch zumindest im Wesentlichen vollflächig mit dem elektrisch leitfähigen Garn bestickt sein.

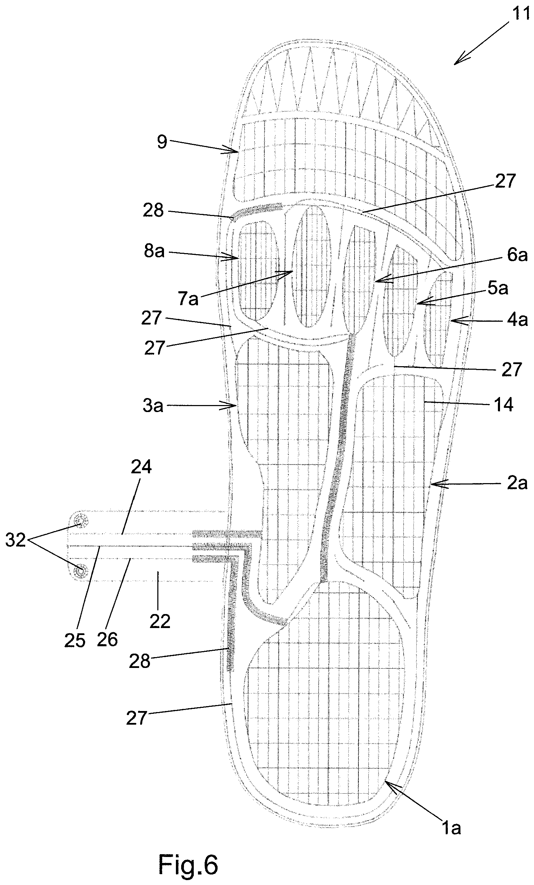

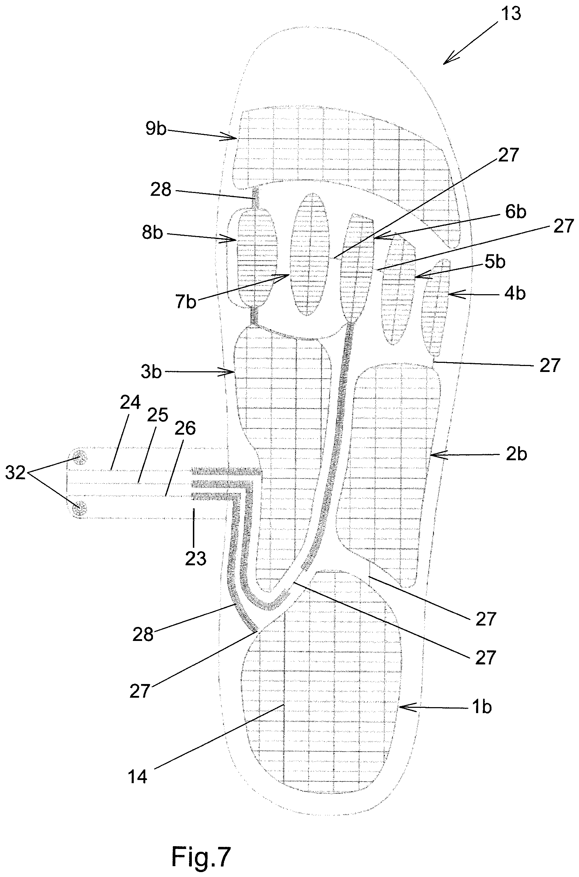

Das elektrisch leitfähige Material 14 ist somit auf der Oberseite der unteren Lage 11 angeordnet (Fig. 6) sowie auf der Unterseite der oberen Lage 13 (in Fig. 7 ist hierzu, damit das elektrisch leitfähige Material 14 sichtbar ist, das Basismaterial der oberen Lage 13 durchsichtig dargestellt).

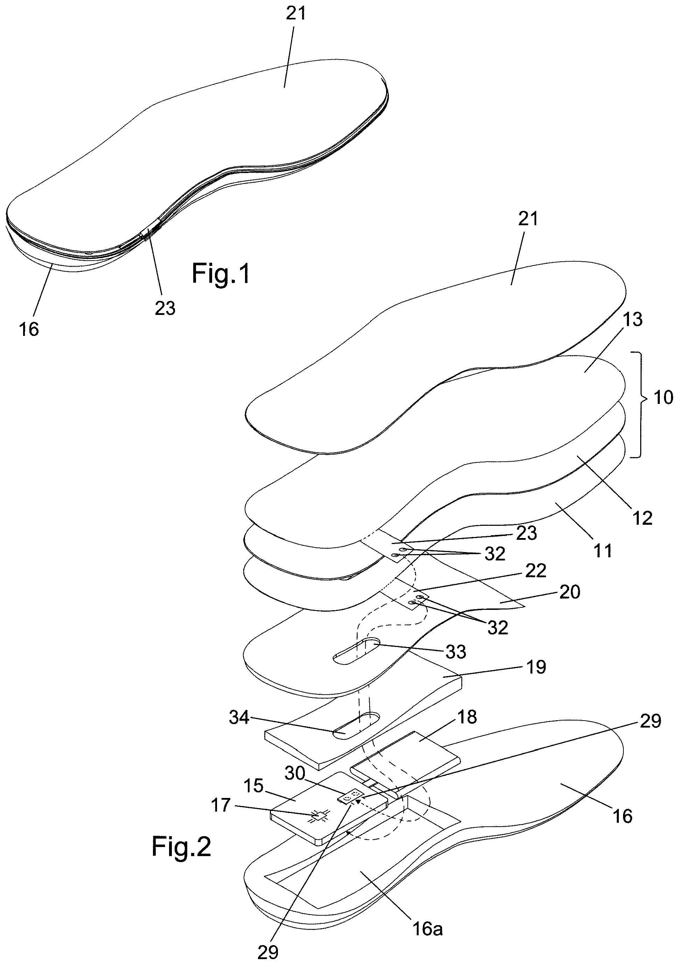

In Fig. 2 ist von der unteren Lage 11 und oberen Lage 13 der Einfachheit halber nur das Basismaterial dargestellt, nicht aber das leitfähige Material 14.

Beim Basismaterial handelt es sich vorzugsweise um ein textiles Material, z.B. kann das Basismaterial von einem Polyestergewebe gebildet werden.

Ein jeweiliges Sensorfeld 1-9 umfasst somit den jeweiligen Bereich 1a-9a der unteren Lage 11, den darüber liegenden Bereich 1b-9b der oberen Lage 13 und den dazwischenliegenden Abschnitt der mittleren Lage 12. Die einem jeweiligen Sensorfeld 1-9 zugeordneten Bereiche 1a-9a; 1b-9b liegen also übereinander und sind in Draufsicht gesehen vorzugsweise zumindest im Wesentlichen deckungsgleich.

Die Sensoreinrichtung 10 ist mit einer Elektronikeinheit 15 elektrisch leitend verbunden. Die Elektronikeinheit 15 ist in einer Ausnehmung 16a eines Sohlenbasisteils 16 angeordnet. Die Elektronikeinheit 15 weist elektronische Bauteile 17 auf, von denen lediglich in Fig. 2 eines angedeutet ist. Insbesondere handelt es sich bei der Elektronikeinheit 15 um eine Platine, die mit den elektronischen Bauteilen 17 bestückt ist.

Zur Energieversorgung der Elektronikeinheit 15 dient ein Akkumulator 18. Dieser ist ebenfalls in der Ausnehmung 16a des Sohlenbasisteils 16 angeordnet und mit der Elektronikeinheit 15 elektrisch verbunden.

Das Sohlenbasisteil 16 kann beispielsweise aus Kork ausgebildet sein.

Zwischen dem Sohlenbasisteil 16 und der Sensoreinrichtung 10 können weitere Lagen, insbesondere Dämpfungs- und/oder Isolierungslagen angeordnet sein. Im Ausführungsbeispiel ist auf dem Sohlenbasisteil im Bereich der Ausnehmung 16a eine Dämpfungslage 19, beispielsweise aus Kunststoff, zur Dämpfung von Stößen angeordnet.

Zwischen dem Sohlenbasisteil 16 und der Sensoreinrichtung 10 ist im Ausführungsbeispiel - günstigerweise oberhalb der Dämpfungslage 19, soweit eine solche vorhanden ist - eine Stabilisierungslage 20 angeordnet, welche sich zumindest über einen hinteren Teil des Sohlenbasisteils 16 erstreckt. Durch diese vorteilhafterweise vorgesehene Stabiliserungslage 20 wird eine zu starke Durchbiegung des Sohlenbasisteils 16 im Bereich der Elektronikeinheit 15 und/oder des Akkumulators 18 verhindert. Die Stabilisierungslage 20 kann aus Kunststoff bestehen.

Oberhalb der Sensoreinrichtung 10 kann wie dargestellt eine Lage 21 zur Fußauflage vorgesehen sein, beispielsweise aus einem textilen Material.

Die verschiedenen Lagen 16, 19, 20, 11, 12, 13, 24 der Einlegesohle sind miteinander verbunden, insbesondere durch Verkleben und/oder Vernähen.

Zur elektrischen Verbindung der Sensorfelder 1-9 mit der Elektronikeinheit 15 weisen die untere Lage 11 und die obere Lage 13 jeweils eine abstehende flexible Lasche 22, 23 auf. Eine jeweilige Lasche 22, 23 ist mit Leiterbahnen 24, 25, 26 versehen. Die über die Laschen 22, 23 verlaufenden Leiterbahnen 24-26 setzen sich über die untere Lage 11 und obere Lage 13 fort und bilden Sensorzuleitungen 27, um das elektrisch leitfähige Material 14 der betreffenden Bereiche 1a-9a; 1b-9b mit den Leiterbahnen 24, 25, 26 der Laschen 22, 23 zu verbinden.

Im Ausführungsbeispiel sind neun Sensorfelder 1-9 vorhanden und somit neun elektrisch leitfähiges Material 14 aufweisende Bereiche 1a-9a; 1b-9b der unteren Lage 11 sowie der oberen Lage 13, und sowohl die Lasche 22 der unteren Lage 11 als auch die Lasche 23 der oberen Lage 13 weist drei Leiterbahnen 24-26 auf. Speziell bestehen hierbei folgende Verbindungen:
Die Leiterbahn 24 der Lasche 22 der unteren Lage 11 ist mit dem elektrisch leitfähigen Material 14 in den Bereichen 3a, 7a, 4a verbunden, die Leiterbahn 25 der Lasche 22 mit dem elektrisch leitfähigen Material 14 in den Bereichen 1a, 6a und 8a und die Leiterbahn 26 der Lasche 22 mit dem elektrisch leitfähigen Material 14 der Bereiche 2a, 5a und 9a. Die Leiterbahn 24 der Lasche 23 der oberen Lage 13 ist mit dem elektrisch leitfähigen Material 14 in den Bereichen 3b, 8b, 9b verbunden, die Leiterbahn 25 der Lasche 23 mit dem elektrisch leitfähigen Material 14 in den Bereichen 5b, 6b und 7b und die Leiterbahn 26 der Lasche 23 mit dem elektrisch leitfähigen Material 14 in den Bereichen 1b, 2b und 4b.

So kann zwischen einer der Leiterbahnen 24-26 der Lasche 22 der unteren Lage und einer der Leiterbahnen 24-26 der Lasche 23 der oberen Lage 13 jeweils der elektrische Widerstand genau eines der Sensorfelder 1-9 gemessen werden, beispielsweise zwischen der Leiterbahn 24 der Lasche 22 und der Leiterbahn 24 der Lasche 23 der elektrische Widerstand des Sensorfeldes 3.

Die Leiterbahnen 24-26 der unteren und oberen Lasche 22, 23 und die Sensorzuleitungen 27 der unteren Lage 11 und oberen Lage 13 werden im gezeigten Ausführungsbeispiel durch ein elektrisch leitfähiges Garn ausgebildet, mit welchem das Grundmaterial der jeweiligen Lasche 22, 23 bzw. der jeweiligen Lage 11, 13 bestickt ist.

Im Bereich von Überkreuzungspunkten zwischen Sensorzuleitungen 27 der unteren und oberen Lage 11, 13 sind die Sensorzuleitungen 27 elektrisch isoliert. Hierzu wird im gezeigten Ausführungsbeispiel das elektrisch leitfähige Garn im zu isolierenden Bereich mit einem elektrisch isolierenden Garn 28, z.B. einem Polyestergarn, überstickt.

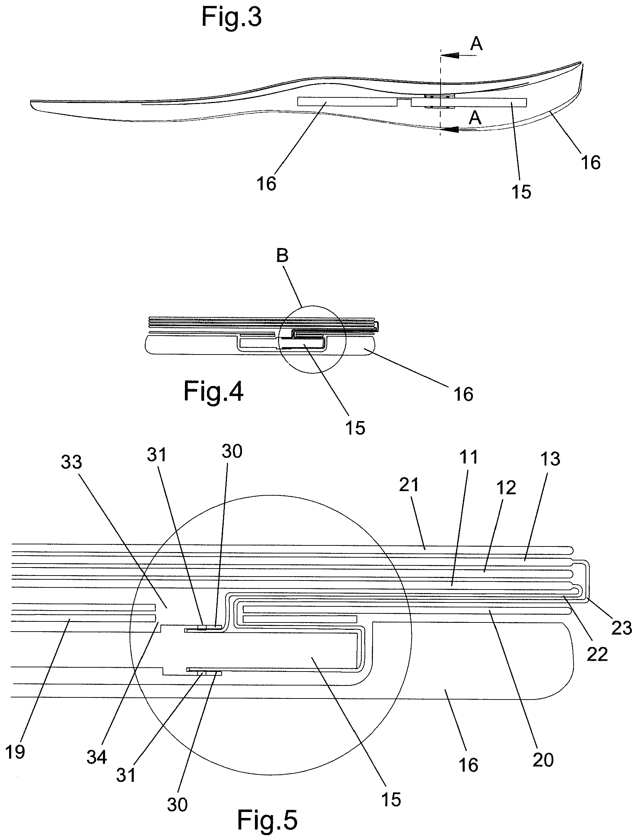

Die Laschen 22, 23 sind vorzugsweise materialeinstückig mit dem Grundmaterial der jeweiligen Lage 11, 13 ausgebildet. Obwohl in der schematisierten Fig. 5 die Laschen 22, 23 mit einer geringeren Materialstärke als die jeweilige Lage 11, 13 dargestellt sind, könnten die Laschen 22, 23 die gleiche Materialstärke wie die jeweilige Lage 11, 13 aufweisen, wie dies bevorzugt ist.

Zur Ausbildung der unteren Lage 11 bzw. oberen Lage 13 mit der jeweils daran angeordneten Lasche 22, 23 kann somit ein flächiges, insbesondere textiles, Grundmaterial mit elektrisch leitfähigem Garn bestickt werden, um die Bereiche 1a-9a; 1b-9b, die Leiterbahnen 24-26 der Laschen 22, 23 und die Sensorzuleitungen 27 auszubilden. Auszubildende Isolierungen können, wie bereits erwähnt, durch Besticken mit einem elektrisch isolierenden Garn hergestellt werden. In der Folge kann die jeweilige Lage 11, 13 mit der daran angeordneten Lasche 22, 23 zugeschnitten werden. Auf diese Weise können die untere Lage und die obere Lage insgesamt durch textile Herstellungsverfahren ausgebildet werden.

Im flach ausgelegten Zustand der jeweiligen Lage 11, 13 mit der daran angeordneten Lasche 22, 23 steht die Lasche 22, 23 seitlich von der Lage 11, 13 ab und liegt in der gleichen Ebene mit dieser. Die Laschen 22, 23 könnten auch als Fahnen bezeichnet werden.

Zur Ausbildung der Sensoreinrichtung 10 werden die untere Lage 11, die mittlere Lage 12 und die obere Lage 13 übereinander gelegt und miteinander verbunden, beispielsweise durch Vernähen.

Das elektrisch leitfähige Garn zur Ausbildung der Bereiche 1a-9a; 1b-9b der unteren Lage 11 und oberen Lage 13 und/oder der Leiterbahnen 24, 26 der Laschen 22, 23 und/oder der Sensorzuleitungen 27 kann ein Multifilgarn oder Monofilgarn sein. Beispielsweise kann es sich um ein Edelstahl-Garn handeln, welches mehrere 100 Filamente aufweist, z.B. mit einer Dicke im Bereich von 5 µm bis 15 µm. Das elektrisch leitfähige Garn kann auch durch Stapelfasern ausgebildet sein.

Zum Besticken der jeweiligen Lage 11, 13 und/oder Lasche 22, 23 mit dem elektrisch leitfähigen Garn kann dieses elektrisch leitfähige Garn den Hinterfaden bilden, wobei der Vorderfaden von einem elektrisch isolierenden Garn gebildet werden kann, z.B. Polyester, oder umgekehrt.

Um die Sensoreinrichtung 10 mit der Elektronikeinheit 15 elektrisch zu verbinden, werden die Laschen 22, 23 umgelegt (um eine parallel zur Mittelebene durch die jeweilige Lage 11 und parallel zur Längserstreckung der Sohle liegende Achse umgebogen bzw. umgeknickt) und an Kontaktbereiche 29 der Elektronikeinheit 15 angelegt. Nur in Fig. 2 sind zwei solche Kontaktbereiche 29 an der Oberseite der Elektronikeinheit 15 schematisch angedeutet. Bei den Kontaktbereichen 29 handelt es sich insbesondere um metallisierte Bereiche der Platine der Elektronikeinheit 15.

Um die Leiterbahnen 24-26 der Laschen 22, 23 an die Kontaktbereiche 29 der Elektronikeinheit 15 anzudrücken, dienen im Ausführungsbeispiel Klemmelemente 30. Ein jeweiliges Klemmelement 30 wird von einer federelastischen, an der Elektronikeinheit 15 angebrachten Zunge gebildet. Die Klemmelemente 30 weisen hierbei günstigerweise Positionierungsstifte 31 auf, welche durch Löcher 32 in den Laschen 22, 23 ragen.

Im Ausführungsbeispiel befinden sich die Kontaktbereiche 29 für die Leiterbahnen 24-26 der Lasche 22 der unteren Lage 11 an der Oberseite der Elektronikeinheit 15 und die Kontaktbereiche 29 für die Leiterbahnen 24-26 der Lasche 23 der oberen Lage 13 an der Unterseite der Elektronikeinheit 15. Bei in Draufsicht nicht übereinander liegend angeordneten Leiterbahnen 24-26 der beiden Laschen 22, 23 könnten die Kontaktbereiche 29 für beide Laschen auch an der Oberseite der Elektronikeinheit oder könnten die Kontaktbereiche für beide Laschen auch an der Unterseite der Elektronikeinheit angeordnet sein.

Die Stabilisierungslage 20 und die Dämpfungslage 19 besitzen im Ausführungsbeispiel Fensteröffnungen 33, 34, durch welche die Laschen 22, 23 im mit der Elektronikeinheit 15 verbundenen Zustand durchgeführt sind. Es wäre stattdessen auch denkbar und möglich, die Laschen 22, 23 um diese Lagen 19, 20 außen herum zu führen.

Im Ausführungsbeispiel stehen die flexiblen Laschen 22, 23 im flach ausgelegten Zustand der Sensoreinrichtung 10 (also im noch nicht mit der Elektronikeinheit 15 verbundenen Zustand) seitlich von der Sensoreinrichtung 10 ab, zumindest im Wesentlichen in eine Richtung rechtwinkelig zur Richtung der Längserstreckung der Einlegesohle. Stattdessen könnten die Laschen 22, 23 beispielsweise auch vom vorderen oder hinteren Ende der Sensoreinrichtung 10 abstehen (parallel zur Richtung der Längserstreckung der Einlegesohle), obwohl dies in Hinblick auf die Gefahr einer Beschädigung der Laschen 22, 23 weniger bevorzugt ist.

Zur Übertragung von Daten der Elektronikeinheit 15, beispielsweise an ein Mobilfunkgerät oder eine andere außerhalb der Einlegesohle angeordnete Mikroprozessoreinrichtung kann die Elektronikeinheit 15 vorzugsweise eine Sende- und Empfangseinheit zur drahtlosen Datenübermittlung aufweisen, beispielsweise über Bluetooth. Ein Aufladen des Akkumulators 18 kann in einer möglichen Ausführungsform ebenfalls drahtlos erfolgen. Stattdessen oder zusätzlich könnte eine (in den Figuren nicht dargestellte) Anschlussbuchse zur Datenübertragung und/oder zum Aufladen des Akkumulators 18 vorhanden sein.

Mittels einer erfindungsgemäßen Einrichtung kann u.a. der auf die verschiedenen Sensorfelder jeweils einwirkende Druck und dessen zeitlicher Verlauf erfasst werden, z.B. um Fehlbelastungen des Nutzers festzustellen.

Die Bereiche 1a-9a; 1b-9b mit dem elektrisch leitfähigen Material 14 könnten auch in anderer Weise als durch Besticken mit einem elektrisch leitfähigen Garn ausgebildet sein. Beispielsweise könnte die untere und/oder obere Lage 11, 13 in diesen Bereichen auch mit einer Metallbeschichtung versehen sein.

Die Leiterbahnen 24-26 und/oder Sensorzuleitungen 27 könnten auch in anderer Weise als durch Besticken mit einem leitfähigen Material ausgebildet sein, beispielsweise durch Metallbeschichtungen.

Die untere und/oder obere Lage 11, 13 könnte als Grundmaterial anstelle eines textilen Materials auch eine Folie aufweisen (die mit elektrisch leitfähigem Material bestickt ist und/oder mit Metallbeschichtungen versehen ist).

Anstelle des Überstickens mit einem elektrisch isolierenden Garn 28 könnten Isolierungen auch in anderer Weise ausgebildet sein, beispielsweise durch Beschichtung mit einem elektrisch isolierenden Material.

Beim gezeigten Ausführungsbeispiel handelt es sich um eine Einlegesohle für einen Schuh. Die Erfindung kann ebenso bei einer Schuhsohle eines Schuhs eingesetzt werden, welche in analoger Weise wie die dargestellte Einlegesohle ausgebildet sein kann, gegebenenfalls mit einem entsprechend geänderten Schichtaufbau, beispielsweise einer zusätzlichen Untersohle.

**Legende zu den Hinweisziffern:**

| | | | |
|---|---|---|---|
| 1 | Sensorfeld | 10 | Sensoreinrichtung |
| 1a | Bereich | 11 | untere Lage |
| 1b | Bereich | 12 | mittlere Lage |
| 2 | Sensorfeld | 13 | obere Lage |
| 2a | Bereich | 14 | elektrisch leitfähiges Material |
| 2b | Bereich | 15 | Elektronikeinheit |
| 3 | Sensorfeld | 16 | Sohlenbasisteil |
| 3a | Bereich | 16a | Ausnehmung |
| 3b | Bereich | 17 | elektronisches Bauteil |
| 4 | Sensorfeld | 18 | Akkumulator |
| 4a | Bereich | 19 | Dämpfungslage |
| 4b | Bereich | 20 | Stabilisierungslage |
| 5 | Sensorfeld | 21 | Lage |
| 5a | Bereich | 22 | Lasche |
| 5b | Bereich | 23 | Lasche |
| 6 | Sensorfeld | 24 | Leiterbahn |
| 6a | Bereich | 25 | Leiterbahn |
| 6b | Bereich | 26 | Leiterbahn |
| 7 | Sensorfeld | 27 | Sensorzuleitung |
| 7a | Bereich | 28 | elektrisch isolierendes Garn |
| 7b | Bereich | 29 | Kontaktbereich |
| 8 | Sensorfeld | 30 | Klemmelement |
| 8a | Bereich | 31 | Positionierungsstift |
| 8b | Bereich | 32 | Loch |
| 9 | Sensorfeld | 33 | Fensteröffnung |
| 9a | Bereich | 34 | Fensteröffnung |
| 9b | Bereich | | |

## Patentansprüche

1. Einlegesohle oder Schuhsohle, welche eine Sensoreinrichtung (10) mit mehreren Sensorfeldern (1-9) zur Druckerfassung und eine mit den Sensorfeldern (1-9) elektrisch verbundene Elektronikeinheit (15) mit elektronischen Bauteilen (17) aufweist, wobei die Sensoreinrichtung (10) sandwichartig ausgebildet ist mit einer mittleren Lage (12) aus einem piezoresistiven Material, einer oberhalb der mittleren Lage (12) angeordneten oberen Lage (13) und einer unterhalb der mittleren Lage (12) angeordneten unteren Lage (11), wobei die obere und untere Lage (13, 11) im Bereich (1a-9a; 1b-9b) eines jeweiligen Sensorfeldes (1-9) jeweils ein elektrisch leitfähiges Material (14) aufweisen, und die obere und untere Lage (13, 11) zur elektrischen Verbindung der Sensorfelder (1-9) mit der Elektronikeinheit (15) jeweils eine abstehende, flexible Lasche (23, 22) mit Leiterbahnen (24-26) aufweisen, und die Elektronikeinheit (15) Kontaktbereiche (29) aufweist, an welchen die Laschen (23, 22) mit ihren Leiterbahnen (24-26) anliegen, wobei zwischen der Sensoreinrichtung (10) und der Elektronikeinheit (15) eine Stabilisierungslage (20) und/oder Dämpfungslage (19) angeordnet ist.

2. Einlegesohle oder Schuhsohle nach Anspruch 1, **dadurch gekennzeichnet, dass** die Kontaktbereiche (29) für die Leiterbahnen (24-26) der Lasche (22) der unteren Lage (11) an der Oberseite der Elektronikeinheit (15) und die Kontaktbereiche (29) für die Leiterbahnen (24-26) der Lasche (23) der oberen Lage (13) an der Unterseite der Elektronikeinheit (15) angeordnet sind.

3. Einlegesohle oder Schuhsohle nach Anspruch 1 oder 2, **dadurch gekennzeichnet, dass** die Elektronikeinheit (15) eine Platine aufweist, die mit den elektronischen Bauteilen (17) bestückt ist, wobei die Kontaktbereiche (29) von metallisierten Bereichen der die elektronischen Bauteile (17) aufweisenden Platine der Elektronikeinheit (15) gebildet werden.

4. Einlegesohle oder Schuhsohle nach einem der Ansprüche 1 bis 3, **dadurch gekennzeichnet, dass** die Laschen (22, 23) mit ihren Leiterbahnen (24-26) mittels Klemmelementen (30) an die Kontaktbereiche (29) der Elektronikeinheit (15) angedrückt sind.

5. Einlegesohle oder Schuhsohle nach einem der Ansprüche 1 bis 4, **dadurch gekennzeichnet, dass** eine jeweilige Lasche (22, 23) mindestens ein Loch (32) aufweist, durch welches im mit der Elektronikeinheit (15) verbundenen Zustand ein Positionierungsstift (31) der Elektronikeinheit (15) ragt.

6. Einlegesohle oder Schuhsohle nach einem der Ansprüche 1 bis 5, **dadurch gekennzeichnet, dass** die Laschen (22, 23) im mit der Elektronikeinheit (15) verbundenen Zustand durch eine Fensteröffnung (33, 34) in der Stabilisierungslage (20) und/oder Dämpfungslage (19) verlaufen.

7. Einlegesohle oder Schuhsohle nach einem der Ansprüche 1 bis 6, **dadurch gekennzeichnet, dass** die obere und untere Lage (13, 11) jeweils ein flexibles, vorzugsweise textiles, Grundmaterial aufweisen und die Lasche (23) der oberen Lage (13) materialeinstückig mit dem Grundmaterial der oberen Lage (13) und die Lasche (22) der unteren Lage (11) materialeinstückig mit dem Grundmaterial der unteren Lage (11) ausgebildet ist.

8. Einlegesohle oder Schuhsohle nach einem der Ansprüche 1 bis 7, **dadurch gekennzeichnet, dass** das elektrisch leitfähige Material (14), welches die obere Lage (13) und untere Lage (11) im Bereich (1a-9a; 1b-9b) eines jeweiligen Sensorfeldes (1-9) aufweisen, von einem elektrisch leitfähigen Garn gebildet wird, mit welchem ein Grundmaterial der oberen Lage (13) und unteren Lage (11) bestickt ist.

9. Einlegesohle oder Schuhsohle nach einem der Ansprüche 1 bis 8, **dadurch gekennzeichnet, dass** die Leiterbahnen (24-26) der Laschen (22, 23) von einem elektrisch leitfähigen Garn gebildet werden, mit welchem ein Grundmaterial der jeweiligen Lasche (22, 23) bestickt ist.

10. Einlegesohle oder Schuhsohle nach einem der Ansprüche 1 bis 9, **dadurch gekennzeichnet, dass** die Elektronikeinheit (15) in einer Ausnehmung (16a) eines Sohlenbasisteils (16) angeordnet ist und ein zur Energieversorgung der Elektronikeinheit (15) dienender Akkumulator (18) ebenfalls in der Ausnehmung (16a) des Sohlenbasisteils (16) angeordnet und mit der Elektronikeinheit (15) elektrisch verbunden ist.

## Claims

1. An insole or shoe sole, which has a sensor arrangement (10), with a plurality of sensor fields (1-9), for pressure detection and an electronics unit (15), electrically connected to the sensor fields (1-9), with electronic component parts (17), wherein the sensor arrangement (10) is sandwich-like with a middle layer (12) of a piezoresistive material, an upper layer (13) arranged above the middle layer (12) and a lower layer (11) arranged below the middle layer (12), wherein the upper and lower layer (13, 11) each have an electrically conductive material (14) in the region (1a-9a; 1b-9b) of a respective sensor field (1-9), and the upper and lower layer (13, 11) each have a protruding, flexible tab (23, 22), with conducting tracks (24-26), for electrical connection of the sensor fields (1-9) to the electronics unit (15), and the electronics unit (15) has contact regions (29) against which the tabs (23, 22) rest with their conducting tracks (24-26), wherein a stabilization layer (20) and/or damping layer (19) is/are arranged between the sensor arrangement (10) and the electronics unit (15).

2. An insole or shoe sole according to claim 1, **characterized in that** the contact regions (29) for the conducting tracks (24-26) of the tab (22) of the lower layer (11) are arranged at the upper side of the electronics unit (15) and the contact regions (29) for the conducting tracks (24-26) of the tab (23) of the upper layer (13) are arranged at the lower side of the electronics unit (15).

3. An insole or shoe sole according to claim 1 or 2, **characterized in that** the electronics unit (15) has a printed circuit board fitted with the electronic component parts (17), wherein the contact regions (29) are formed by metallised regions of the printed circuit board, having the electronic component parts (17), of the electronics unit (15).

4. An insole or shoe sole according to any one of claims 1 to 3, **characterized in that** the conducting tracks (24-26) of the tabs (22, 23) are pressed against the contact regions (29) of the electronics unit (15) by means of clamping elements (30).

5. An insole or shoe sole according to any one of claims 1 to 4, **characterized in that** a respective tab (22, 23) has at least one hole (32) through which in the state connected to the electronics unit (15) there projects a positioning pin (31) of the electronics unit (15).

6. An insole or shoe sole according to any one of claims 1 to 5, **characterized in that** in the state connected to the electronics unit (15), the tabs (22, 23) pass through a window opening (33, 34) in the stabilization layer (20) and/or damping layer (19).

7. An insole or shoe sole according to any one of claims 1 to 6, **characterized in that** the upper and lower layer (13, 11) each have a flexible, preferably textile, base material and the tab (23) of the upper layer (13) is in a single piece with the base material of the upper layer (13) and the tab (22) of the lower layer (11) is in a single piece with the base material of the lower layer (11).

8. An insole or shoe sole according to any one of claims 1 to 7, **characterized in that** the electrically conductive material (14), which the upper layer (13) and lower layer (11) have in the region (1a-9a; 1b-9b) of a respective sensor field (1-9), is formed by an electrically conductive yarn with which a base material of the upper layer (13) and lower layer (11) is embroidered.

9. An insole or shoe sole according to any one of claims 1 to 8, **characterized in that** the conducting tracks (24-26) of the tabs (22, 23) are formed by an electrically conductive yarn with which a base material of the respective tab (22, 23) is embroidered.

10. An insole or shoe sole according to any one of claims 1 to 9, **characterized in that** the electronics unit (15) is arranged in a recess (16a) of a sole base part (16) and an accumulator (18) serving to supply energy to the electronics unit (15) is likewise arranged in the recess (16a) of the sole base part (16) and is electrically connected to the electronics unit (15).

## Revendications

1. Semelle intérieure amovible ou semelle de chaussure, laquelle est munie d'un dispositif capteur (10) avec plusieurs champs de capteurs (1-9) pour mesurer la pression et d'une unité électronique (15) reliée électriquement aux champs de capteurs (1-9) et munie de composants électroniques (17), le dispositif capteur (10) étant conçu en sandwich avec une couche centrale (12) faite d'un matériau piézorésistant, une couche supérieure (13) disposée au-dessus de la couche centrale (12) et une couche inférieure (11) disposée en dessous de la couche centrale (12), les couches supérieure et inférieure (13, 11) étant munies, dans la zone (1a - 9a ; 1b-9b) d'un champ de capteurs (1-9) respectif, chacune d'un matériau électriquement conducteur (14), et les couches supérieure et inférieure (13, 11) étant munies chacune d'une patte flexible saillante (23, 22) présentant des pistes conductrices (24-26) en vue de relier électriquement les champs de capteurs (1-9) à l'unité électronique (15), et l'unité électronique (15) est munie de zones de contact (29) contre lesquelles les pattes (23, 22) s'appuient avec leurs pistes conductrices (24-26), une couche de stabilisation (20) et/ou une couche d'amortissement (19) étant disposée entre le dispositif capteur (10) et l'unité électronique (15).

2. Semelle intérieure amovible ou semelle de chaussure selon la revendication 1, **caractérisée en ce que** les zones de contact (29) pour les pistes conductrices (24-26) de la patte (22) de la couche inférieure (11) sont disposées sur la face supérieure de l'unité électronique (15) et les zones de contact (29) pour les pistes conductrices (24-26) de la patte (23) de la couche supérieure (13) sont disposées sur la face inférieure de l'unité électronique (15).

3. Semelle intérieure amovible ou semelle de chaussure selon la revendication 1 ou 2, **caractérisée en ce que** l'unité électronique (15) est munie d'une plaquette à circuit imprimé qui est équipée des composants électroniques (17), les zones de contact (29) étant formées par des zones métallisées de la plaquette à circuit imprimé de l'unité électronique (15) qui comporte les composants électroniques (17).

4. Semelle intérieure amovible ou semelle de chaussure selon l'une des revendications 1 à 3, **caractérisée en ce que** les pattes (22, 23) sont pressées avec leurs pistes conductrices (24-26) contre les zones de contact (29) du module électronique (15) au moyen d'éléments de serrage (30).

5. Semelle intérieure amovible ou semelle de chaussure selon l'une des revendications 1 à 4, **caractérisée en ce qu'**une patte (22, 23) respective présente au moins un trou (32) au travers duquel dépasse une broche de positionnement (31) de l'unité électronique (15) à l'état relié à l'unité électronique (15).

6. Semelle intérieure amovible ou semelle de chaussure selon l'une des revendications 1 à 5, **caractérisée en ce que** les pattes (22, 23), à l'état relié à l'unité électronique (15), passent par une ouverture de fenêtre (33, 34) dans la couche de stabilisation (20) et/ou la couche d'amortissement (19).

7. Semelle intérieure amovible ou semelle de chaussure selon l'une des revendications 1 à 6, **caractérisée en ce que** les couches supérieure et inférieure (13, 11) sont munie chacune d'un matériau de base flexible, de préférence textile, et la patte (23) de la couche supérieure (13) est formée d'une pièce avec le matériau de base de la couche supérieure (13), et la languette (22) de la couche inférieure (11) est formée d'une pièce avec le matériau de base de la couche inférieure (11).

8. Semelle intérieure amovible ou semelle de chaussure selon l'une des revendications 1 à 7, **caractérisée en ce que** le matériau électriquement conducteur (14), dont sont munis la couche supérieure (13) et la couche inférieure (11) dans la zone (1a-9a ; 1b-9b) d'un champ de capteurs (1-9) respectif, est formé par un fil électriquement conducteur avec lequel est brodé un matériau de base de la couche supérieure (13) et de la couche inférieure (11).

9. Semelle intérieure amovible ou semelle de chaussure selon l'une des revendications 1 à 8, **caractérisée en ce que** les pistes conductrices (24-26) des pattes (22, 23) sont formées par un fil électriquement conducteur avec lequel est brodé un matériau de base de la patte respective (22, 23).

10. Semelle intérieure amovible ou semelle de chaussure selon l'une des revendications 1 à 9, **caractérisée en ce que** l'unité électronique (15) est disposée dans un évidement (16a) d'une partie de base de semelle (16), et un accumulateur (18) servant à alimenter l'unité électronique (15) en énergie est également disposé dans l'évidement (16a) de la partie de base de semelle (16) et est relié électriquement à l'unité électronique (15).
